(19) Europäisches Patentamt
European Patent Office
Office européen des brevets

(11) Publication number: **0 281 927 B1**

# (12) EUROPEAN PATENT SPECIFICATION

(45) Date of publication of patent specification: **28.06.95** (51) Int. Cl.[6]: **C12Q 1/68**

(21) Application number: **88103221.3**

(22) Date of filing: **03.03.88**

## (54) Assay for nucleic acid sequences in a sample.

(30) Priority: **11.03.87 US 24643**

(43) Date of publication of application:
**14.09.88 Bulletin 88/37**

(45) Publication of the grant of the patent:
**28.06.95 Bulletin 95/26**

(84) Designated Contracting States:
**CH DE ES FR GB GR IT LI NL SE**

(56) References cited:
**EP-A- 0 130 515** **EP-A- 0 133 473**
**EP-A- 0 184 017** **EP-A- 0 187 332**
**EP-A- 0 235 726** **EP-A- 0 237 833**
**US-A- 4 358 535**

**ANALYTICAL BIOCHEMISTRY, vol. 152, no. 2, 01 February 1986, Orlando, FL (US); R.M. AMASINO, pp. 304-307&NUM;**

(73) Proprietor: **Bayer Corporation**
**One Mellon Center**
**500 Grant Street**
**Pittsburgh, PA 15219-2502 (US)**

(72) Inventor: **Dattagupta, Nanibhushan, Dr.**
**470 Prospect Street**
**New Haven, CT 06511 (US)**
Inventor: **Rabin, Daniel, Dr.**
**24 Stone Street**
**Branford, CT 06405 (US)**
Inventor: **Rae, Peter, Dr.**
**71 Ingram Street**
**Hamden, CT 06517 (US)**
Inventor: **Huguenel, Edward, Dr.**
**11 Beech Road**
**Guilford, CT 06437 (US)**

(74) Representative: **Dänner, Klaus et al**
**Bayer AG**
**Konzernzentrale RP**
**Patente Konzern**
**D-51368 Leverkusen (DE)**

EP 0 281 927 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

### Field of the Invention

The present application relates to the detection and identification of microorganisms and the detection and identification of particular prokaryotic or eukaryotic DNA sources in a nucleic acid containing test sample.

Still further, the present invention relates to a method for the lysis of whole cells.

### Background Information

### A. The Detection of Microorganisms

The identification of species of microorganisms in a sample containing a mixture of microorganisms, by immobilizing the DNA from the sample and subjecting it to hybridization with a labelled specimen of species - specific DNA from a known microorganism and observing whether hybridization occurs between the immobilized DNA and the labelled specimen, has been disclosed in PCT patent application No. PCT/US83/01029.

The most efficient and sensitive method of detection of nucleic acids such as DNA after hybridization requires radioactively labeled DNA. The use of autoradiography and enzymes prolongs the assay time and requires experienced technicians.

In EP 235 726 which has been filed prior but published after the priority of the present case a rapid nucleic acid hybridization assay is described. This citation is silent with regard to the accelaration properties of polyethylene glycol. EP 184 017 refers to the enhancement of nucleic acid hybridization by anionic polymers.

U.S.P. 4,358,535 to Falkow et al describe infectious disease diagnosis using labeled nucleotide probes complementary to nucleic acid coding for a characteristic pathogen product.

### B. The Detection of Specific Eukaryotic Sequences

The identification of specific sequence alteration in an eukaryotic nucleic acid sample by immobilizing the DNA from the sample and subjecting it to hybridization with a labeled oligonucleotide and observing whether hybridization occurs between the immobilized DNA and the labeled probe, has been described in European Patent Application EP-A 228 075.

It is known that the expression of a specific gene determines the physical condition of a human being. Adult hemoglobin is a tetrameric association of two alpha and two beta subunits. During embryonic and fetal life, the alpha chains are associated with, successively, gamma and delta chains before the adult beta form predominates. The genes for alpha hemoglobin are located on human chromosome number 16 and the genes for gamma, delta and beta hemoglobin are tandemly linked on human chromosome 11. Hemoglobinopathies are heritable diseases that are the result of alterations in the structure of one or more of the hemoglobin genes. Many of the mutations have been characterized in considerable molecular detail and can range from single base pair changes to wholesale deletion of a gene family. For example, a change in the beta-globin gene coding sequence from GAG to GTG at the sixth amino acid position produces sickle-beta-globin and a homozygote can have a disease known as sickle cell anemia. Similarly deletion of particular sequences from alpha-globin or beta-globin genes can cause thalassemias. A recent survey, The New Genetics and Clinical Practice, D. J. Weatherall, The Nuffield Provincial Hospitals Trust, (1982), chapter 2 describes the frequency and clinical spectrum of genetic diseases.

Problems associated with genetic defects can be diagnosed by nucleic acid sequence information. The easiest way to detect such sequence information is to use the method of hybridization with a specific probe of a known sequence.

U.S.P. 4,395,486 to Wilson et al describe a method for the direct analysis of sickle cell anemia using a restriction endonuclease assay.

Edward M. Rubin and Yuet Wai Kan, "A Simple Sensitive Prenatal Test for Hydrops Fetalis Caused By $\alpha$-Thalassaemia", The Lancet, January 12, 1985, pp. 75-77 describes a dot blot analysis to differentiate between the genotypes of homozygous alpha-thalassemia and those of the haemoglobin-H disease and alpha-thalassemia trait.

The most efficient and sensitive method of detection of nucleic acids, such as DNA, after hybridization requires radioactively labelled DNA. The use of autoradiography and enzymes prolongs the assay time and requires experienced technicians.

Recently, a non-radioactive method of labelling DNA was described by Ward et al, European Patent Application 63,879. Ward et al, use the method of nick translation to introduce biotinylated U (uracil) residues into DNA, replacing T (thymine). The biotin residue is then assayed with antibiotin antibody or an avidin-containing system. The detection in this case is quicker than autoradiography, but the nick translation method requires highly skilled personnel. Moreover, biotinylation using biotinylated UTP (uridine triphosphate) works only for thymine-containing polynucleotides. The use of other nucleoside triphosphates is very difficult because the chemical derivatization of A (adenine) or G (guanine) or C (cytosine) (containing $-NH_2$) with biotin requires the skills of trained organic chemists.

C. Effect of Nonionic Polymers on Hybridization

It has been shown by Wetmur (Biopolymers, 14, 2517-2524, (1974)) that anionic polymers such as dextran sulfate increase the rate of DNA-DNA hybridization in solution. It has also been shown that the heterogeneous phase hybridization rate can also be increased by dextran sulfate. U.S. Patent 4,302,204 discloses the effect of charged polysaccharides on the rate of nucleic acid hybridization.

Recently Amasino (Analytical Biochemistry, 152, 304-307 (1986)) has shown that a neutral polymer like polyethylene glycol can increase the rate of DNA-RNA hybridization more than dextran sulfate under optimum conditions. Although polyethylene glycol (PEG) has been used before in the hybridization medium (e.g., Renz and Kurz, Nucleic Acids Res., 12, 3435-3444 (1984)), no advantage was predicted over the use of dextran sulfate. Applicants have observed, to their surprise, that in the presence of 10% polyethylene glycol hybridization can be virtually finished in 15 minutes. Although Amasino supra has shown PEG is better than dextran sulfate as an accelerator, his labeled probe was single stranded RNA. In the case of the present invention, the labeled material is whole genomic sample DNA where both complementary strands are present in solution. When a polymer accelerates the rate of hybridization it should also help the reannealing in solution, and hence should reduce the efficiency of hybridization, instead of increasing it.

D. Cell Lysis

The present invention also provides a method for the efficient lysis of whole cells such that their DNA is released and made available for photochemical labeling. While eukaryotic cells derived from multicellular animals are easily lysed under relatively mild conditions, single cell eukaryotes and prokaryotes, especially Gram positive prokaryotes, are more difficult to lyse due to the complicated chemical nature and extent of cross-linking of their cell walls. Methods do exist for efficiently lysing these refractory organisms, either by chemical-enzymatic or physical means, but these methods are often complicated, time-consuming and inappropriate for preserving the integrity of DNA.

SUMMARY OF THE INVENTION

The invention concerns a rapid and convenient method for detection of one or more polynucleotide sequences from microorganisms or eucaryotic sources in a biological sample comprising the use of polyethylene glycol as a hybridization accelerator.

This object and advantage is realized in accordance with the present invention for a method of detecting (i) one or more microorganisms or (ii) nucleic acid sequences from a prokaryotic source or an eukaryotic source in a nucleic acid-containing test sample.

The method involves the following:

(a) labeling the nucleic acids, e.g., the nucleic acids of the organisms or cells or cell debris, in the test sample,

(b) contacting, under hybridization conditions, the labeled hybridizable sample nucleic acid and one or more immobilized hybridizable (e.g., single-stranded) nucleic acid (e.g., DNA) probes comprising of (i) one or more known microorganisms or (ii) nucleic acid sequences from eukaryotic or prokaryotic sources, to form hybridized labeled nucleic acids and

(c) assaying for the hybridized nucleic acids by detecting the label wherein the hybridization is accelerated by the addition of polyethylene glycol.

The hybridizable nucleic acid can be whole genomic nucleic acid or a fragment thereof, e.g., an oligonucleotide.

In step (a), the nucleic acids can be labeled directly in the test sample. In step (a), in such direct labeling, cells of the test sample can be lysed and then a labeling reagent can be added, whereby to label the nucleic acid.

The method further comprises denaturing the labeled nucleic acids from step (a) to form labeled denatured nucleic acids.

The labeled nucleic acid test sample is contacted simultaneously with several different types of nucleic acid, e.g., DNA, probes for hybridization. The nucleic acid test sample is labeled and hybridized with several unlabeled immobilized probes. The positions of the probes are fixed, and the labeled probe detected after hybridization will indicate that the test sample carries a nucleic acid sequence complementary to the corresponding probe.

Nucleic acid probes for several microbiological systems or for different alleles of one or more genes can be immobilized separately on a solid support, for example, nitrocellulose paper. The test sample nucleic acids are labeled and remain in solution. The solid material containing the immobilized probe is brought in contact with the labeled test nucleic acid solution under hybridization conditions. The solid material is washed free of unhybridized nucleic acid and the label is assayed. The presence of the label with one or more of the probes indicates that the test sample contains nucleic acids substantially complementary to those probes and hence originate, for example, from an infection by particular microbiological systems.

Labeling can be accomplished in a whole living cell or a cell lysate, and can be non-isotopic. The nucleic acid can be used for hybridization without further purification.

As mentioned above the present invention relates to a hybridization medium which accelerates the process of hybridization. The hybridization accelerator according to the present invention is polyethylene glycol.

The present invention further concerns a method of hybridization comprising contacting two complementary single stranded nucleic acids under hybridization conditions, at least one of which is immobilized wherein polyethylene glycol is added.

## DETAILED DESCRIPTION OF THE INVENTION

The test sample in the present invention includes body fluids, e.g., urine, blood, semen, cerebrospinal fluid, pus, amniotic fluid, tears, or semisolid or fluid discharge, e.g., sputum, saliva, lung aspirate, vaginal or urethal discharge, stool or solid tissue samples, such as a biopsy or chorionic villispecimens. Test samples also include samples collected with swabs from the skin, genitalia, or throat.

All these test samples can be used for hybridization diagnosis after labeling the nucleic acids of the sample. The labeling can be accomplished without any processing of the sample. As for example, a urine sample (suspected of bacterial infections) can be labeled without centrifugation, filtration or dialysis. The cells in the samples can be lysed without any separation step. It is surprising that a nucleic acid labeling reaction takes place in such a complex mixture as a clinical sample.

The nucleic acid is preferably labeled by means of photochemistry, employing a photoreactive DNA-binding furocoumarin or a phenanthridine compound to link the nucleic acid to a label which can be "read" or assayed in conventional manner, including fluorescence detection. The end product is thus a labeled nucleic acid comprising (a) a nucleic acid component, (b) an intercalator or other DNA-binding ligand photochemically linked to the nucleic acid component, and (c) a label chemically linked to (b).

The photochemical method provides more favorable reaction conditions than the usual chemical coupling method for biochemically sensitive substances. The intercalator and label can first be coupled and then photoreacted with the nucleic acid, or the nucleic acid can first be photoreacted with the intercalator and then coupled to the label.

A general scheme for coupling a nucleic acid, exemplified by double-stranded DNA, to apply a label, is as follows:

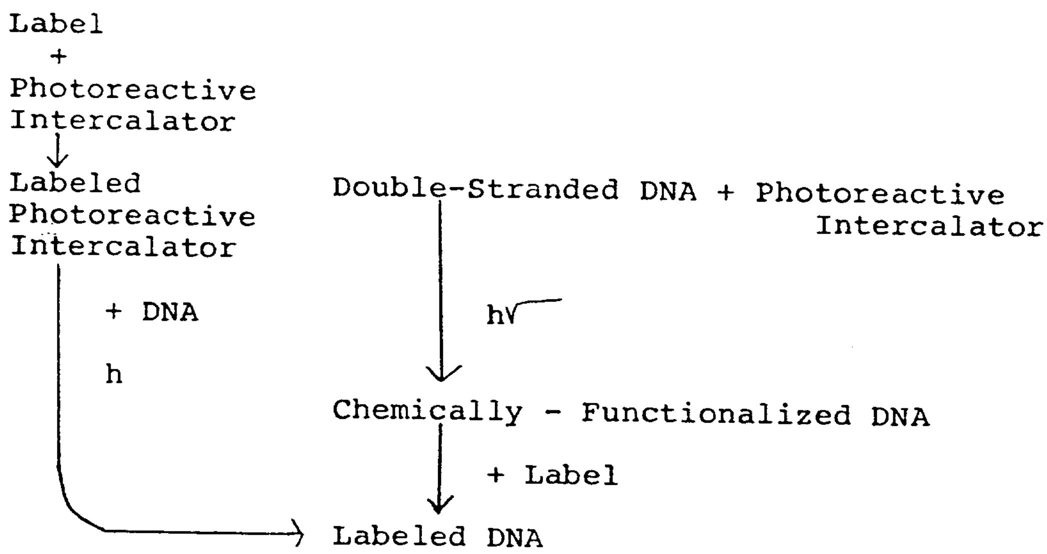


Where the hybridizable portion of the nucleic acid is in a double stranded form, such portion is then denatured to yield a hybridizable single stranded portion. Alternatively, where the labeled DNA comprises the hybridizable portion already in single stranded form, such denaturation can be avoided if desired.

To produce specific and efficient photochemical products, it is desirable that the nucleic acid component and the photoreactive intercalator compound be allowed to react in the dark in a specific manner.

For coupling to DNA, aminomethyl psoralen, aminomethyl angelicin and amino alkyl ethidium or methidium azides are particularly useful compounds. They bind to double-stranded DNA and only the complex yields photoadduct. In the case where labeled double-stranded DNA must be denatured in order to yield a hybridizable single stranded region, conditions are employed so that simultaneous interaction of two strands of DNA with a single photoadduct is prevented. It is necessary that the frequency of modification along a hybridizable single stranded portion of the nucleic acid not be so great as to substantially prevent hybridization, and accordingly there preferably will be not more than one site of modification per 25, more usually 50, and preferably 100, nucleotide bases. Angelicin derivatives are superior to psoralen compounds for monoadduct formation. If a single-stranded DNA nucleic acid is covalently attached to some extra double-stranded DNA, use of phenanthridium and psoralen compounds is desirable since these compounds interact specifically to double-stranded DNA in the dark. The chemistry for the synthesis of the coupled reagents to modify nucleic acids for labeling, described more fully hereinbelow, is similar for all cases.

The nucleic acid component can be single or double stranded DNA or RNA or fragments thereof such as are produced by restriction enzymes or even relatively short oligomers.

The DNA-binding ligands used to link the nucleic acid component to the label can be any suitable photoreactive form of known DNA-binding ligands. Particularly preferred DNA-binding ligands are intercalator compounds such as the furocoumarins, e.g., angelicin (isopsoralen) or psoralen or derivatives thereof which photochemically will react with nucleic acids, e.g., 4'-aminomethyl-4,5'-dimethyl angelicin, 4'-aminomethyl-trioxsalen (4'aminomethyl-4,5',8-trimethyl-psoralen), 3-carboxy-5- or -8-amino-or-hydroxy-psoralen, as well as mono- or bis-azido aminoalkyl methidium or ethidium compounds.

Particularly useful photoreactive forms of intercalating agents are the azidointercalators. Their reactive nitrenes are readily generated at long wavelength ultraviolet or visible light and the nitrenes of arylazides prefer insertion reactions over their rearrangement products (see White et al, Methods in Enzymol., 46, 644 (1977)). Representative intercalating agents include azidoacridine, ethidium monoazide, ethidium diazide, ethidium dimer azide (Mitchell et al, JACS, 104, 4265 (1982)), 4-azido-7-chloroquinoline, and 2-azidofluorene. A specific nucleic acid binding azido compound has been described by Forster et al, Nucleic Acid Res., 13, (1985), 745. The structure of such compound is as follows:

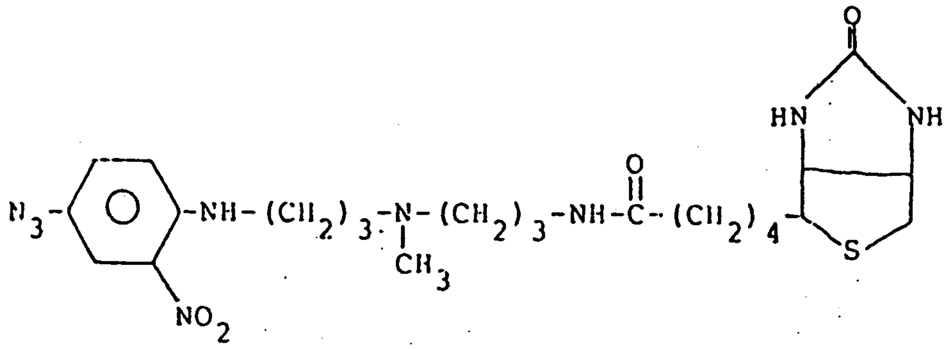

Other useful photoreactable intercalators are the furocoumarins which form (2+2) cycloadducts with pyrimidine residues. Alkylating agents can also be used such as bis-chloroethylamines and epoxides or aziridines, e.g., aflatoxins, polycyclic hydrocarbon epoxides, mitomycin and norphillin A.

Nonlimiting examples of intercalator compounds for use in the present invention include acridine dyes, phenanthridines, phenazines, furocoumarins, phenothiazines and quinolines.

The label which is linked to the nucleic acid component according to the present invention can be any chemical group or residue having a detectable physical or chemical property, i.e., labeling can be conducted by chemical reaction or physical adsorption. The label will bear a functional chemical group to enable it to be chemically linked to the intercalator compound. Such labeling materials have been well developed in the field of immunoassays and in general most any label useful in such methods can be applied to the present invention. Particularly useful are enzymatically active groups, such as enzymes (see Clin. Chem., (1976), 22, 1243), enzyme substrates (see British Pat. Spec. 1,548,741), coenzymes (see U.S. Patent Nos. 4,230,797 and 4,238,565) and enzyme inhibitors (see U.S. Patent No. 4,134,792; fluorescers (see Clin. Chem., (1979), 25, 353), and chromophores including phycobiliproteins; luminescers such as chemiluminescers and bioluminescers (see Clin. Chem., (1979), 25, 512, and ibid, 1531); specifically bindable ligands, i.e., protein binding ligands; and residues comprising radioisotopes such as $^{3}H$, $^{35}S$, $^{32}P$, $^{125}I$, and $^{14}C$. Such labels are detected on the basis of their own physical properties (e.g., fluorescers, chromophores and radioisotopes) or their reactive or binding properties (e.g., enzymes, substrates, coenzymes and inhibitors). For example, a cofactor-labeled nucleic acid can be detected by adding the enzyme for which the label is a cofactor and a substrate for the enzyme. A hapten or ligand (e.g., biotin) labeled nucleic acid can be detected by adding an antibody or an antibody pigment to the hapten or a protein (e.g., avidin) which binds the ligand, tagged with a detectable molecule. An antigen can also be used as a label. Such detectable molecule can be some molecule with a measurable physical property (e.g, fluorescence or absorbance) or a participant in an enzyme reaction (e.g., see above list). For example, one can use an enzyme which acts upon a substrate to generate a product with measurable physical property. Examples of the latter include, but are not limited to, beta-galactosidase, alkaline phosphatase, papain and peroxidase. For in situ hybridization studies, ideally the final product is water insoluble. Other labels, e.g., dyes, will be evident to one having ordinary skill in the art.

The label will be linked to the intercalator compound, e.g., acridine dyes, phenanthridines, phenazines, furocoumarins, phenothiazines and quinolines, by direct chemical linkage such as involving covalent bonds, or by indirect linkage such as by the incorporation of the label in a microcapsule or liposome which in turn is linked to the intercalator compound. Methods by which the label is linked to the intercalator compounds are essentially known in the art and any convenient method can be used to perform the present invention.

Advantageously, the intercalator compound is first combined with label chemically and thereafter combined with the nucleic acid component. For example, since biotin carries a carboxyl group, it can be combined with a furocoumarin by way of amide or ester formation without interfering with the photochemical reactivity of the furocoumarin or the biological activity of the biotin, e.g.,

(i)

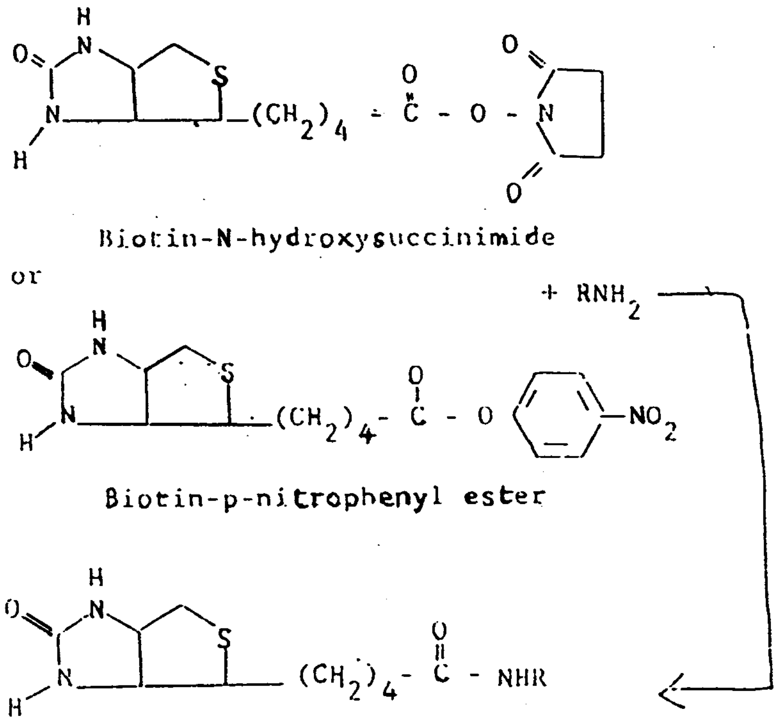

Biotin-N-hydroxysuccinimide

or

+ $RNH_2$

(ii)

Biotin-p-nitrophenyl ester

(iii)

or

$$\text{Biotin} + \text{ROH} \xrightarrow{\text{carbodiimide}} \text{Biotin CO OR}$$

By way of example,

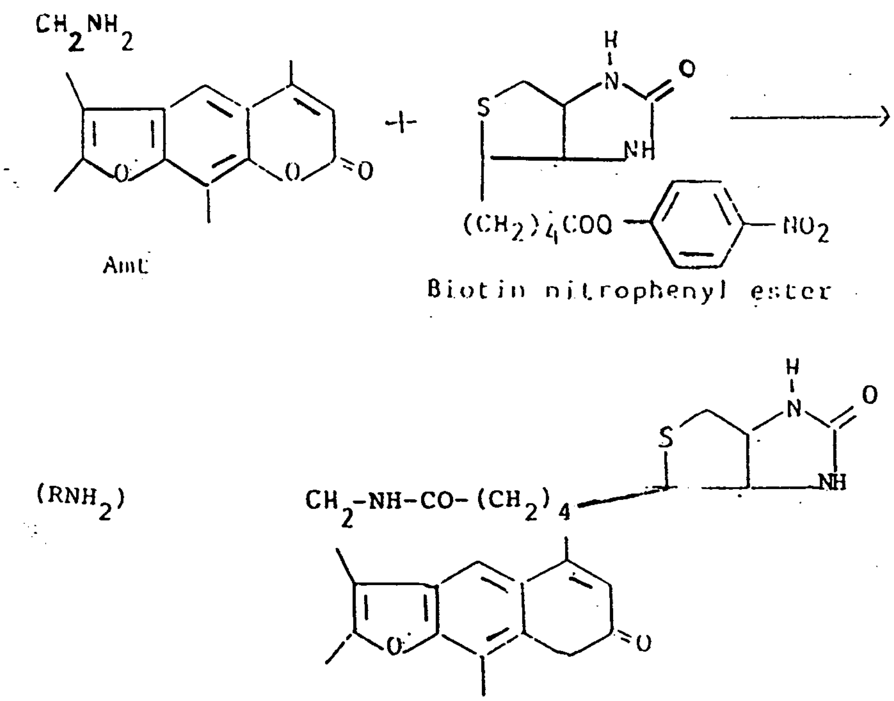

Amt

Biotin nitrophenyl ester

($RNH_2$)

Other aminomethylangelicin, psoralen and phenanthridium derivatives can be similarly reacted, as can phenanthridium halides and derivatives thereof such as aminopropyl methidium chloride, i.e.,

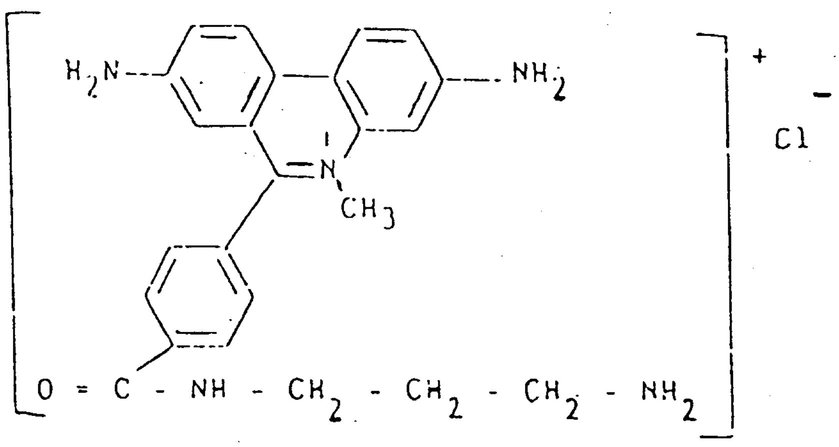

(see Hertzberg et al, J. Amer. Chem. Soc., 104, 313 (1982)).

Alternatively, a bifunctional reagent such as dithiobis succinimidyl propionate or 1,4-butanediol diglycidyl ether can be used directly to couple the photochemically reactive molecule with the label where the reactants have alkyl amino residues, again in a known manner with regard to solvents, proportions and reaction conditions. Certain bifunctional reagents, possibly glutraldehyde may not be suitable because, while they couple, they may modify nucleic acid and thus interfere with the assay. Routine precautions can be taken to prevent such difficulties.

The particular sequence used in making the labeled nucleic acid can be varied. Thus, for example, an amino-substituted psoralen can first be photochemically coupled with a nucleic acid, the product having pendant amino groups by which it can be coupled to the label, i.e., labeling can be carried out by photochemically reacting a DNA binding ligand with the nucleic acid in the test sample. Alternatively, the psoralen can first be coupled to a label such as an enzyme and then to the nucleic acid.

As described in European Patent Application EP-A 187 332 the present invention also encompasses a labeled nucleic acid comprising (a) a nucleic acid component, (b) a nucleic acid-binding ligand photochemically linked to the nucleic acid component, (c) a label and (d) a spacer chemically linking (b) and (c).

Advantageously, the spacer includes a chain of up to about 40 atoms, preferably about 2 to 20 atoms, selected from the group consisting of carbon, oxygen, nitrogen and sulfur.

Such spacer may be the polyfunctional radical of a member selected from the group consisting of peptide, hydrocarbon, polyalcohol, polyether, polyamine, polyimine and carbohydrate, e.g., -glycyl-glycyl-glycyl-or other oligopeptide, carbonyl dipeptides, and omega-amino-alkane-carbonyl radical such as $-NH(CH_2)_5-CO-$, a spermine or spermidine radical, an alpha, omega-alkanediamine radical such as $-NH-(CH_2)_6-NH$ or $-HN-CH_2-CH_2-NH$, or the like. Sugar, polyethylene oxide radicals, glyceryl, pentaerythritol, and like radicals can also serve as the spacers.

These spacers can be directly linked to the nucleic acid-binding ligand and/or the label or the linkages may include a divalent radical of a coupler such as dithiobis succinimidyl propionate, 1,4-butanediol diglycidyl ether, a diisocyanate, carbodiimide, glyoxal, glutaraldehyde, or the like.

The spacer can be incorporated at any stage of the process of making the probe.

a-b-d-c

defined hereinabove. Thus, the sequence can be any of the following:

a + b + d + c,
b + d + c + a,
d + c + b + a,
b + d + a + c, etc.

The conditions for the individual steps are well known in chemistry.

If the label is an enzyme, for example, the product will ultimately be placed on a suitable medium and the extent of catalysis will be determined. Thus, if the enzyme is a phosphatase, the medium could contain nitrophenyl phosphate and one would monitor the amount of nitrophenol generated by observing the color.

If the enzyme is a beta-galactosidase, the medium can contain o-nitro- phenyl-D-galacto-pyranoside which also will liberate nitrophenol.

The labeled nucleic acid of the present invention is applicable to all conventional hybridization assay formats, and in general to any format that is possible based on formation of a hybridization product or aggregate comprising the labeled nucleic acid. In particular, the unique labeled nucleic acid of the present invention can be used in solution and solid-phase hybridization formats; including, in the latter case, formats involving immobilization of either sample or probe nucleic acids and sandwich formats.

The nucleic acid probe will comprise at least one hybridizable, e.g., single-stranded, base sequence substantially complementary to or homologous with the sequence to be detected. However, such base sequence need not be a single continuous polynucleotide segment, but can be comprised of two or more individual segments interrupted by nonhomologous sequences. These nonhomologous sequences can be linear or they can be self-complementary and form hairpin loops. In addition, the homologous region of the probe can be flanked at the 3' - and 5' termini by nonhomologous sequences, such as those comprising the DNA or RNA or a vector into which the homologous sequence had been inserted for propagation In either instance, the probe as presented as an analytical reagent will exhibit detectable hybridization at one or more points with sample nucleic acids of interest. Linear or circular hybridizable, e.g., single-stranded polynucleotides can be used as the probe element, with major or minor portions being duplexed with a complementary polynucleotide strand or strands, provided that the critical homologous segment or segments are in single-stranded form and available for hybridization with sample DNA or RNA. Useful probes include linear or circular probes wherein the homologous probe sequence is in essentially only single-stranded form (see particularly, Hu and Messing, Gene, 17:271 (1982)).

In solid-phase hybridization formats, one of the polynucleotide species participating in hybridization is fixed in an appropriate manner in its single-stranded form to a solid support. Useful solid supports are well known in the art and include those which bind nucleic acids either covalently or noncovalently. Noncovalent supports which are generally understood to involve hydrophobic bonding include naturally occurring and synthetic polymeric materials, such as nitrocellulose, derivatized nylon and fluorinated polyhydrocarbons, in a variety of forms such as filters, beads or solid sheets. Covalent binding supports (in the form of filters, beads or solid sheets, just to mention a few) are also useful and comprise materials having chemically reactive groups or groups, such as dichlorotriazine, diazobenzyloxymethyl, and the like, which can be activated for binding to polynucleotides.

It is well known that noncovalent immobilization of an oligonucleotide is ineffective on a solid support, for example, on nitrocellulose paper. The present invention also describes novel methods of oligonucleotide immobilization. This is achieved by phosphorylation of an oligonucleotide by a polynucleotide kinase or by ligation of the 5'-phosphorylated oligonucleotide to produce multi-oligonucleotide molecules capable of immobilization. The conditions for kinase and ligation reaction have been described in standard text books, e.g., Molecular Cloning, T. Maniatis, E.F. Fritsch and J. Sambrook, Cold Spring Harbor Laboratory, (1982), pages 1-123.

For the present invention, the immobile phase of the hybridization system can be a series or matrix of spots of known kinds and/or dilutions of denatured DNA. This is most simply prepared by pipetting appropriate small volumes of native DNA onto a dry nitrocellulose or nylon sheet, floating the sheet on a sodium hydroxide solution to denature the DNA, rinsing the sheet in a neutralizing solution, then baking the sheet to fix the DNA. Before DNA:DNA hybridization, the sheet is usually treated with a solution that inhibits non-specific binding of added DNA during hybridization.

This invention involves the labeling of whole genomic DNA, whole nucleic acids present in cells, whole cell lysate, or unlysed whole cells. Once the labeled material is prepared, it can be used for the detection, i.e., the presence or absence of certain specific genomic sequences by specific nucleic acid hybridization assays.

One method according to the invention involves the separation of cells from a human sample or the human sample directly is treated by mixing with a photochemically reactive nucleic acid binding intercalating ligand. The mixture is incubated depending on the type of the sample. If the sample is lysed cells or nucleic acids, it is incubated for a period between a few seconds to five minutes and when whole cells or partially lysed cells are used, incubation between two minutes to two hours is employed. After the mixing and incubation, the whole sample mixture is irradiated at a particular wavelength for the covalent interaction between the photochemically reactive DNA binding ligand and the test sample. Then this labeled material is hybridized under specific hybridization conditions with a specific probe.

After the hybridization, the unreacted unhybridized labeled test sample is removed by washing. After the washing, the hybrid is detected through the label carried by the test sample, which is specifically hybridized with a specific probe.

The present invention is surprising since in a human genomic sample the amount of a single copy gene is very low, for example, if a restriction fragment of one thousand base pairs is the region of hybridization, the frequency of such sequence in the whole human genomic sample is one in a million. This conclusion has been derived by assuming from the literature that a human genomic sample has $3 \times 10^9$ base pairs and 1000 base pairs will be 1/3,000,000 of that number. Automatically, in a sample of human DNA containing approximately five to ten micrograms of nucleic acids, only 5 to 10 picogram of the corresponding sequences is available and labeling the vast majority of the non-specific DNA should produce more background than the true signal. But after the reaction, it is surprising to observe that the results are not only specific, but also of unexpected higher sensitivity.

Without wishing to be bound by any particular theory of operability, the reason for the unexpected sensitivity may be due to the formation of a network of non-specific nucleic acid hybrids bound to the specific hybrid, thus amplifying the amount of the signal. As has been shown in a typical example, a 19 nucleotide long specific sequence containing plasmid is immobilized and hybridized with 5 microgram equivalent of a test sample which is labeled photochemically and one detects very easily the signal resulted from such hybrid. This could not have been accomplished by any other technique because of the problems associated with the labeling method.

The present invention relates to a novel hybridization technique where probes are immobilized and an eukaryotic nucleic acid sample is labeled and hybridized with immobilized unlabeled probe. A surprising characteristic of the invention is the ability to detect simple or multiple copy gene defects by labeling the test sample. Since there is no requirement for an excess of labeled hybridizing sequence, the present method is more specific. In the present invention, simultaneous detection of different gene defects can be easily carried out by immobilizing specific probes.

For example, using the present invention, one can immobilize oligonucleotide probes specific for genetic defects related to hemoglobinopathines, such as sickle cell anemia and alpha-thalassemias on a sheet of nitrocellulose paper, label the test sample and hybridize the labeled test sample with the immobilized probes. It is surprising that partially purified or unpurified nucleic acid samples (cell lysate or whole cell) can be photochemically labeled with sensitive molecules without affecting the specific hybridizability.

The oligonucleotide can be cloned in cloning vectors, e.g., M 13, PUC 19 and PBR and accordingly the vector containing the oligonucleotide acts as the probe.

The present invention is also directed to detecting eukaroytes (protists) in samples from higher organisms, such as animals or humans.

Eukaroytes include algae, protozoa, fungi and slime molds.

The term "algae" refers in general to chlorophyll-containing protists, descriptions of which are found in G.M. Smith, Cryptogamic Botany, 2nd ed. Vol. 1, Algae and Fungi, McGraw-Hill, (1955).

Eukaryotic sequences according to the present invention includes all disease sequences except for bacteria and viruses. Accordingly, genetic diseases, for example, would also be embraced by the present invention. Non-limiting examples of such genetic diseases are as follows:

| Area Affected | Diseases |
| --- | --- |
| Metabolism | Acute intermittent porphyria |
| | Variegate porphyria |
| | $alpha_1$-antitrypsin deficiency |
| | Cystic fibrosis |
| | Phenylketonuria |
| | Tay-Sachs disease |
| | Mucopolysaccharidosis I |
| | Mucopolysaccharidosis II |
| | Galactosaemia |
| | Homocystinuria |
| | Cystinuria |
| | Metachromic leucodystrophy |
| Nervous System | Huntington's chorea |
| | Neurofibromatosis |
| | Myotonic dystrophy |
| | Tuberous sclerosis |
| | Neurogenic muscular atrophies |
| Blood | Sickle-cell anaemia |
| | Beta-thalassaemia |
| | Congenital spherocytosis |
| | Haemophilia A |

| | |
|---|---|
| Bowel | Polyposis coli |
| Kidney | Polycystic disease |
| Eyes | Dominant blindness<br>Retinoblastoma |
| Ears | Dominant early childhood deafness<br>Dominant otosclerosis |
| Circulation | Monogenic hypercholesterolaemia |
| Blood | Congenital spherocytosis |
| Teeth | Dentinogenisis imperfecta<br>Amelogenisis imperfecta |
| Skeleton | Diaphysial aclasia<br>Thanatophoric dwarfism<br>Osteogenes imperfecta<br>Marfan syndrome<br>Achondroplasia<br>Ehlers-Danlos syndrome<br>Osteopetrosis tarda<br>Cleft lip/palate |
| Skin | Ichthyosis |
| Locomotor | Muscular dystrophy |

A nucleic acid probe in accordance with the present invention is a sequence which can determine the sequence of a test sample. The probes are usually DNA, RNA, mixed copolymers of ribo- and deoxyribonucleic acids, oligonucleotides containing ribonucleotides or deoxyribonucleotide residues or their modified forms. The sequence of such a probe should be complementary to the test sequence. The extent of complementary properties will determine the stability of the double helix formed after hybridization. The probe can also have covalently linked non-complementary nucleic acids. They can serve as the sites of the labeling reaction.

The nucleic acid is preferably labeled by means of photochemistry, employing a photoreactive DNA-binding furocoumarin or a phenanthridine compound to link the nucleic acid to a label which can be "read" or assayed in conventional manner, including fluorescence detection.

One use of the present invention is the identification of bacterial species in biological fluids. In one application, samples of urine from subjects having or suspected of having urinary tract infections can provide material for the preparation of labeled DNA(s) or RNAs, while a solid support strip, e.g., made of nitrocellulose or nylon, can contain individual dots or spots of known amounts of denatured purified DNA

from each of the several bacteria likely to be responsible for infection.

The format of labeled unknown and unlabeled probes, which is the converse of standard schemes, allows one to identify among a number of possibilities the species of organism in a sample with only a single labeling. It also allows simultaneous determination of the presence of more than one distinguishable bacterial species in a sample (assuming no DNA in a mixture is discriminated against in the labeling procedure). However, it does not allow in a simple way, better than an estimate of the amount of DNA (and, therefore, the concentration of bacteria) in a mixed sample. For such quantitation, sample DNA is immobilized in a series of dilution spots along with spots of standard DNA, and probe DNAs are labeled.

A urinary tract infection is almost always due to monoclonal growth of one of the following half dozen kinds of microorganism: Escherichia coli (60-90% of UTI), Proteus spp. (5-20% of UTI), Klebsiella spp (3-10% of UTI), Staphylococcus spp. (4-20% of UTI), Streptococcus spp. (2-5% of UTI). Pseudomonas and some other gram negative rods together account for a low percentage of UTI. A common contaminant of urine samples that is a marker of improper sample collection is Lactobacillus.

The concentration of bacteria in a urine sample that defines an infection is about $10^5$ per milliliter.

The format for an unlabeled probe hybridization system applicable to urinary tract infections is to have a matrix of DNAs from the above list of species, denatured and immobilized on a support such as nitrocellulose, and in a range of amounts appropriate for concentrations of bacterial DNAs that can be expected in samples of labelled unknown.

Standard hybridization with biotinylated whole genome DNA probes takes place in 5-10 ml, at a probe concentration of about 0.1 µg/ml: hybridization of probe to a spot containing about 10 ng denatured DNA is readily detectable. There is about 5 fg of DNA per bacterial cell, so that for a sample to contain 1 µg of labeled DNA, it is necessary to collect 2 x $10^8$ bacteria. If an infection produces urine having approximately $10^5$ bacteria/ml, then bacterial DNA to be labeled from a sample is concentrated from 2000 ml. If more than 10 ng unlabeled probe DNA is immobilized in a dot, for example, 100 ng or 1 µg, or if the hybridization volume is reduced, then the volume of urine required for the preparation of labeled unknown is approximately a few tenths of a ml.

A strip of dots containing immobilized, denatured, unlabelled probe DNAs could have the following configuration:

| | 1 µg | 10 ng | 100 pg |
|---|---|---|---|
| Escherichia | o | o | o |
| Proteus | o | o | o |
| Klebsiella | o | o | o |
| Staphylococcus | o | o | o |
| Streptococcus | o | o | o |
| Pseudomonas | o | o | o |
| Lactobacillus | o | o | o |

This procedure involves the labeling of DNA or RNA in a crude cell lysate. Ideally, preparation of labeled sample DNA or RNA will accommodate the following points:

(1) bacteria will be concentrated from a fluid sample by centrifugation or filtration;

(2) bacteria will be lysed under conditions sufficient to release nucleic acids from the most refractory of the organisms of interest;

(3) the labeling protocol will not require purification of labeled nucleic acids from unincorporated precursors, nor the purification of nucleic acids prior to labeling;

(4) the labeling protocol will be sufficiently specific for DNA and/or RNA that proteins, lipids and polysaccharides in the preparation will not interfere with hybridization nor read-out.

In the present invention, there is provided a method for efficiently and rapidly lysing whole cells, including Gram positive bacteria. The method involves contacting cells, e.g., whole cells, with an alkali, e.g., sodium or potassium hydroxide solution in a concentration of 0.1 to 1.6 Normal.

The important features of the present lysis protocol are its relative simplicity and speed. It employs a common chemical that requires no special storage conditions and it lyses even Gram positive organisms with high efficiency, while preserving the properties of the DNA that are important for subsequent steps in the photochemical labeling process.

For the present invention, the immobile phase of the hybridization system can be a series or matrix of spots of known kinds and/or dilutions of denatured DNA. This is most simply prepared by pipetting appropriate small volumes of native DNA or oligonucleotides onto a dry nitrocellulose or nylon sheet,

floating the sheet on a sodium hydroxide solution to denature the DNA, rinsing the sheet in a neutralizing solution, then baking the sheet to fix the DNA. Before DNA:DNA hybridization, the sheet is usually treated with a solution that inhibits non-specific binding of added DNA during hybridization.

The invention will be further described in the following non-limiting examples wherein parts are by weight unless otherwise expressed.

Examples

Example 1: Preparation of Labeling Compound

The preparation of the labeling compound required 1-amino-17-N-(Biotinylamido)-3,6,9,12,15 pentaoxaheptadecane. This compound was prepared in the following four steps:

(a) 3,6,9,12,15 pentaoxaheptadecane 1,17-diol ditosylate was synthesized.
(b) 1,17-dipthalimido derivative of 3,6,9,12, 15 pentaoxaheptadecane was prepared.
(c) 1,17-diamino derivative of 3,6,9,12,15 pentaoxaheptadecane was prepared.
(d) 1-amino, 17-biotinylamido derivative of 3,6,9,12,15 pentaoxaheptadecane was prepared.

Example 1(a): Preparation of 3,6,9,12,15-Pentaoxaheptadecane-1,17-diol Ditosylate

To a stirred solution containing 50 g of hexaethylene glycol (0.177 mol) and 64 ml of triethylamine (39.36 g, 0.389 mol) in 400 ml of $CH_2Cl_2$ at 0°C was added dropwise a solution containing 73.91 g of p-toluenesulfonyl chloride (0.389 mol) in 400 ml of $CH_2Cl_2$ over a 2.5 hour period. The reaction mixture was then stirred for one hour at 0°C and then heated to ambient temperature for 44 hours. The mixture was then filtered and the filtrate was concentrated in vacuo. The resulting heterogeneous residue was suspended in 500 ml of ethyl acetate and filtered. The filtrate was then concentrated in vacuo to a yellow oil which was triturated eight times with 250 ml portions of warm hexane to remove unreacted p-toluenesulfonyl chloride. The resulting oil was then concentrated under high vacuum to yield 108.12 g of a yellow oil (quantitative yield).

| Analysis: Calculated for $C_{26}H_{38}O_{11}S_2$ | | |
|---|---|---|
| Calc.:<br>found: | C, 52.87;<br>C, 52.56; | H, 6.48.<br>H, 6.39. |

PMR: (60 MHz, $CDCl_3$) δ: 2.45 (s, 6H); 3.4-3.8 (m, 20H); 4.2 (m, 4H); 7.8 (AB quartet, J=8Hz, 8H).
IR: (neat) $cm^{-1}$: 2870, 1610, 1360, 1185, 1105, 1020, 930, 830, 785, 670.

Example 1 (b): Preparation of 1,17-Diphthalimido-3,6,9,12,15-pentaoxaheptadecane

A stirred suspension containing 108 g of 3,6,9,12,15-pentaoxaheptadecane-1,17-diol ditosylate (0.183 mol), 74-57 g of potassium phthalimide (0.403 mol), and 700 ml of dimethylacetamide was heated at 160-170°C for 2 hours and was then cooled to room temperature. The precipitate was filtered and washed with water and acetone to yield 53.05 g of product as a white powder which was dried at 55°C (0.1 mm). mp 124-126°C.

A second crop of product was obtained from the dimethylacetamide filtrate by evaporation in vacuo and the resulting precipitate with was successively washed ethyl acetate, water, and acetone. The resulting white powder was dried at 55°C (0.1 mm) to yield an additional 9.7 g of product. mp 124.5-126.5°C. The combined yield of product was 62.82 g (68% yield).

Analysis: (For first crop)
Calculated for $C_{28}H_{32}N_2O_9 \cdot 1/2H_2O$
Calc.: C, 61.19; H, 6.05; N, 5.09.
found: C, 61.08; H. 6.15; N, 5.05.

(For second crop)
Calculated for $C_{28}H_{32}N_2O_9$
Calc.: C, 62.21; H, 5.97; N, 5.18.
found: C, 61.78; H, 6.15; N, 5.13.

PMR: (60 MHz, dmso-$d_6$) δ: 3.5 (s, 8H); 3.6 (s, 8H); 3.8 (bt, J = 3Hz, 8H): 8.1 (s, 8H).
IR: (KBr) $cm^{-1}$: 2890, 1785, 1730, 1400, 1100, 735.

Example 1(c): Preparation of 1,17-Diamino-3,6,9,12,15-Pentaoxaheptadecane

A solution containing 60 g of 1,17-diphthalimido-3,6,9,12,15-pentaoxaheptadecane (0.118 mol), 14.8 g of hydrazine hydrate (0.296 mol), and 500 ml of ethanol were heated with mechanical stirring in a 100 °C oil bath for three hours. The mixture was then cooled and filtered. The resultant filter cake was washed four times with 300 ml portions of ethanol. The combined filtrates were concentrated to yield 32.35 g of a yellow apaque glassy oil. The evaporative distillation at 150-200 °C (0.01 mm) gave 22.82 g of a light yellow oil (69% yield). lit. b.p. 175-177 °C (0.07 mm).

PMR: (60 MHz, $CDCl_3$) δ: 1.77 (s, 4H, $NH_2$); 2.85 (t, J = 5Hz, 4H); 3.53 (t, J = 5Hz, 4H); 3.67 (m, 16H).
IR: ($CHCl_3$) $cm^{-1}$: 3640, 3360, 2860, 1640, 1585, 1460, 1350, 1250, 1100, 945, 920, 870.
Mass Spectrum: (EI) m/e = 281.2 (0.1%, M + 1). (FAB) m/e = 281.2 (100%, M + 1).

| Analysis: For $C_{12}H_{28}N_2O_5 \cdot 1/2\ H_2O$ | | | |
|---|---|---|---|
| Calc.:<br>found: | C, 49.80,<br>C, 50.36; | H, 10.10;<br>H, 9.58; | N, 9.68.<br>N, 9.38. |

Literature Reference: W. Kern, S. Iwabachi, H. Sato and V. Bohmer, Makrol. Chem., 180, 2539 (1979).

Example 1(d): Preparation of 1-Amino-17-N-(Biotinylamido)-3,6,9,12,15-pentaoxaheptadecane

A solution containing 7.2 g of 1,17-diamino-3,6,9,12,15-pentaoxaheptadecane (25 mmol) in 75 ml of DMF under an argon atmosphere was treated with 3.41 g of N-succinimidyl biotin (10 mmol) added in portions over 1.0 hour. The resulting solution was stirred for four hours at ambient temperature. TLC ($SiO_2$, 70:10.1 $CHCL_3$-$CH_3OH$-conc. $NH_4OH$) visualized by dimethylazninocinnamaldehyde spray reagent showed excellent conversion to a new product (Rf = 0.18). The reaction mixture was divided in half and each half was absorbed onto $SiO_2$ and flash-chromatographed on 500 g of $SiO_2$-60 (230-400 mesh) using a 70:10.1 $CHCl_3$-$CH_3OH$-conc. $NH_4OH$ solvent mixture. Fractions containing the product were polled and concentrated to a yield 2.42 g of a gelatinous, waxy solid. The product was precipitated as a solid from isopropanol-ether, washed with hexane, and dried at 55 °C (0.1 mm) to give 1.761 g of a white powder (35% yield).

| Analysis: Calculated for $C_{22}H_{42}N_4O_7S.3/2\ H_2O$: | | | |
|---|---|---|---|
| | C, 49.51; | H, 8.50; | N. 10.49. |
| found: | C, 49.59; | H, 8.13; | N, 10.39. |

PMR: (90 MHz, dmso-$d_6$) δ: 1.1-1.7 (m, 6H); 2.05 (t, J=7Hz, 2H); 2.62 (t, J=4Hz, 1H); 2.74 (t, J=4Hz, 1H); 3.0-3.4 (m, 14H). 3.50 (s, 14H); 4.14 (m, 1H); 4.30 (m, 1H); 6.35 (d, J=4Hz, 1H); 7.80 (m, 1H).

CMR: (22.5 MHz, dmso-$d_6$) δ: 25.2, 28.0, 28.2, 35.1, 40.6, 55.3, 59.2, 61.1, 69.6, 69.8, 71,2, 162.7, 172.1.

IR: (KBr) $cm^{-1}$: 2900, 2850, 1690, 1640, 1580, 1540, 1450, 1100.

Mass Spectrum (FAB) m/e: 507.3 (M+1, 56%)

Example 2: Preparation of 4'-Biotinyl-PEG-4,5'-dimethylangelicin

A solution of 203 mg of 1-amino-17-N-(biotinylamido)-3,6,9.12,15-pentaoxaheptadecane (0.4 mmol) in 1 ml of DMF under an argon atmosphere was treated with 78 mg of N,N-carbonyldimidazole (0.48 mmol). The resulting mixture was stirred for four hours and was then treated with 55 mg of 4'-aminomethyl-4,5'dimethylingelicin hydrochloride (0.2 mmol), 140 µl of diisopropylethylamine, and 100 µl of DMF. The resulting mixture was stirred overnight at 50°C. The mixture was then evaporated onto $SiO_2$ in vacuo and the resultant impregnated solid flash was chromatographed on 60 g of $SiO_2$ (230-400 mesh) eluted with 1.5 liters of 7% $CH_3$-$CHCl_3$ followed by 1 liter of 10% $CH_3OH$-$CHCl_3$. Fractions containing the product were pooled and concentrated to yield 72 mg of a glassy solid (47% yield).

PMR: (90 MHz, dmso-$d_6$): δ 1.1-1.8 (m, 6H); 2.04 (bt, J=7Hz, 2H); 2.5 (s, 6H); 2.56 (m, 1H); 2.74 (bd, J=4Hz, 1H); 2.8-3.4 (m, 14H); 3.40 (m, 14H); 4.14 (m, 1H); 4.25 (m, 1H); 4.40 (bd, J=6Hz, 2H); 6.5 (m, 1H); 6.35 (s, 1H); 7.02 (s, 1H); 7.45 (d, J=8Hz, 1H); 7.62 (d, J=8Hz, 1H); 7.80 (m, 1H).

CMR: (22.5 MHz, dmso-$d_6$) δ: 11.9, 18.9, 25.3, 28.2 28.3, 33.4, 35.2, 55.4, 59.2, 61.0, 69.2, 69.6, 69.8, 70.0, 89.0, 107.8, 112.0, 113.1, 114.3, 120.6, 121.6, 153.6, 154.4, 155.6. 157.9, 159.5, 162.7, 172.1.

Literature Reference: F. Dall'Acqua, D. Vedaldi, S. Caffieri, A. Guiotto, P. Rodighiero, F. Baccichetti, F. Carlassare and F. Bordin, J. Med. Chem., 24, 178 (1981).

Example 3: General Method of Labeling the Test sample Nucleic Acids

High molecular weight DNA from a patient's sample is isolated by a method described in U.S.P. 4,395,486 (Wilson et al). The nucleic acid is dissolved in 10 mM borate buffer (pH 8.0) to a final concentration of approximately 20 µg/ml. To the nucleic acid solution "angelicin-peg-biotin" in aqueous solution is added to a final concentration of 10 µg/ml. The mixture is then irradiated at long wavelength irradiation for about 60 minutes using a black ray UVL 56 lamp. The product is ready for hybridization without purification. However, the product can be purified by dialysis or alcohol precipitation (U.S.P. 4,395,486) as is usually followed for nucleic acids.

Instead of nucleic acids, whole cell lysate can also be labeled following an identical procedure. The lysis is conducted by boiling the cells with 0.1 N sodium hydroxide, followed by neutralization with hydrochloric acid.

When whole cells are used, the mixture of "PEG-ang-bio" and cells are incubated for at least 60 minutes prior to irradiation for efficient transport of the ligands. Many different variations of the above described methods can be adopted for labeling.

Example 4: Hybridization with labeled genomic DNA for Non Radioactive Detection

Human normal genomic (XX) DNA was photolabeled with "biotin-PEG-angelicin" (BPA) in 10 mM borate buffer pH 8.2 at a weight ratio of 0.3 to 1 (BPA:DNA) for 15 minutes on ice with a long wave UV lamp model UVL-21, λ = 366 nm. No purification is necessary.

Target DNA oligonucleotides were directly immobilized on S & S nitrocellulose in 1 µl aliquots at the following concentration, and then baked in an 80°C vacuum oven for 30 minutes. The amounts of the different immobilized probes are as follows:

| | | | |
|---|---|---|---|
| 43-mer | (A) | - Kinased (method 1) | 200 ng |
| 43-mer | (A) | | 200 ng |
| 43-mer | (S) | - Kinased (method 1) | 200 ng |
| 43-mer | (S) | | 200 ng |
| M1319Ass | | | 50 ng |
| M1319Sss | | | 50 ng |
| M13737Ass | | | 50 ng |

| | |
|---|---|
| BRL Commercially biotinylated DNA | 200 pg |
| pUC19 | 50 ng |

43-merA: 5' $GGAT_3AAT_4CTCCTG\overset{*}{A}GGAGAAGTCT$
$GCT_4AATCTTAA$ 3'

* =T for 43-mer S
16-mer (Common to both A and S)
3' - TTAGAATTCCTAAATT-5'

Filters were prehybridized in blotto (5% nonfat dry milk, 6XSSC, 20 mM Na-pyrophosphate) for 30 minutes in a 45 °C $H_2O$ bath.

All 4 strips were hybridized in 2 mls solution containing 2 µg labeled XX DNA containing normal beta-globin gene (hybridization solution was blotto with 10% PEG) for 2 hours in 45 °C in a $H_2O$ bath.

A stringency wash was carried out as follows:

1 X 20′ at room temperature in 6XSSC

2 X 20′ at temperatures indicated in Fig. 1 with very little agitation.

50 ml centrifuge tubes were used for elevated temperature washes. Results are shown in Fig. 1.

Detection of biotin in the hybrid was carried out according to the Bethesda Research Laboratory, Bethesda, Maryland, U.S.A., manual using their kit for biotin detection. The results indicated specific hybridization.

Example 5: Detection of Urinary Tract Infection in a Urine Sample

Urine samples were collected from a hospital where they were analyzed by microbiological methods and the results were kept secret until the hybridization diagnosis was conducted. Then they were compared ascertain the validity of the hybridization results.

1 ml of clinical sample (urine) suspected of UTI infection was centrifuged in a Brinkman micro centrifuge for 5 minutes. Then 0.1 ml of 1.2 N sodium hydroxide was added and the suspension was heated to 100 °C to lyse the cells. The suspension was then diluted to 1 ml with 10 mM sodium borate buffer pH 8 and was neutralized with hydrochlorine acid to a pH of 7. To the solution, 50 µg "biotin-PEG-angelicin" (see Example 2) is added and the mixture was irradiated with a UVL 56 long wavelength UV lamp for 15 minutes. The irradiated sample (0.1 ml) was added to 3 ml 3XSSC of 5% nonfat dry milk 10% PEG with 0.2 M sodium pyrophosphate and hybridization was conducted with probes (whole genomic DNA) immobilized onto nitrocellulose paper at 68 °C for 5 minutes to overnight. After hybridization detection was conducted with chemoluminescence, with colorimetric method or immunogold, the spots or the photographs were visually interpreted for the presence of specific bacteria in the test sample. A spot of human DNA was also present in the nitrocellulose paper for the detection of leucocytes. The presence of leucocytes was further verified with a common method using "LEUKOSTIX"® (Miles Laboratories, Elkhart, Indiana, U.S.A.).

Typical results (Tables 1 and 2) indicate that the hybridization diagnosis produces similar results in a shorter time then the corresponding microbiological assays. The present invention not only provides information related to species identification, but also the leucocyte content in a clinical sample.

TABLE 1

DIAGNOSIS OF CLINICAL URINE SAMPLES

| * HOSPITAL DIAGNOSIS | APPLICANTS' HYBRIDIZATION RESULTS | DETECTION SYSTEM |
|---|---|---|
| NEG | NEG | GOLD |
| NEG | NEG | GOLD |
| NEG | NEG | GOLD |
| NEG | E.c.-M | CHEMI |
| NEG | E.c.-VW | GOLD |
| NEG | E.c.-VW | GOLD |
| S+, C- | NEG | GOLD |
| S+, C- | E.c.-S | CHEMI |
| S+, C- | E.c.-S, Kl.-M | CHEMI |
| S+, C- | NEG | GOLD |
| S+, C- | NEG | GOLD |
| S+, C- | NEG | GOLD |
| S+, C- | E.c.-VW | GOLD |
| S+, C- | NEG | GOLD |
| S+, C- | E.c.-VW | GOLD |
| S+, C- | NEG | GOLD |
| S+, C- | NEG | GOLD |
| 100,000/mL E.c. | E.c.-S | GOLD |
| 100,000/mL E.c. | E.c.-S | CHEMI |
| 100,000/mL E.c. | E.c.-W | GOLD |
| 50,000/mL E.c. | E.c.-M | CHEMI |
| 50,000/mL E.c. | NEG | GOLD |
| E. coli | E.c.-S, Kl.-M | CHEMI |
| E. coli | E.c.-VS, Kl.-S | CHEMI |
| E. coli | E.c.-S, Kl.-S | CHEMI |
| E. coli/Klebsiella mix | E.c.-S, Kl.-W | GOLD |
| E. coli/Staph mix | E.c.-S, St.-M | CHEMI |

TABLE 1 (Continued)

DIAGNOSIS OF CLINICAL URINE SAMPLES

| * HOSPITAL DIAGNOSIS | APPLICANTS' HYBRIDIZATION RESULTS | DETECTION SYSTEM |
|---|---|---|
| Klebsiella spp. | E.c.-M, Kl.-W | CHEMI |
| 100,000/mL K. pneumoniae | E.c.-W, Kl-VW | GOLD |
| Enterobacter spp. | NEG** | GOLD |
| 100,000 Candida | NEG** | GOLD |
| 100,000/mL Proteus | Pr.-S, E.c.-W | GOLD |
| 10,000/mL Strep | NEG | CHEMI |
| Mixture of 3 unidentified Gm(+) | NEG | GOLD |

* diagnosis conducted by streaking urine on an agar plate and treating the plate under conditions so that the infectious organism can grow.

** Enterobacter/Candida probes not included in the hybridization assay, therefore, negative results are not surprising; given the high stringency conditions employed in the assay, cross-hybridization with species related to Enterobacter was not detected.

Abbreviations: VS=very strong; S=strong; M=medium; W=weak; and VW=very weak hybridization signals; GOLD=detection method according to Example 12; CHEMI=chemiluminescent detection according to Example 3(b)

Applicants' hybridization results represent the result of a subjective interpretation of the intensity of the hybridization signals obtained after detection. DNAs from the organisms listed in column two are the only ones for which any hybridization signal was obtained. The panel of DNAs used for hybridization included E. coli ("E.c."), Klebsiella pneumoniae ("Kl"), Proteus vulgaris ("Pr"), Pseudomonas aeruginosa, Staphylococcus epidermatis ("SE"), Streptococcus faecalis and Homo sapiens.

TABLE 2

COMPARISON OF AMES LEUKOSTIX ASSAY WITH APPLICANT'S ASSAY

| "LEUKOSTIX" RESULT | APPLICANTS' HYBRIDIZATION RESULT | DETECTION SYSTEM |
|---|---|---|
| 3+ | VS | GOLD |
| 3+ | S | CHEMI |
| 3+ | S | CHEMI |
| 3+ | M | CHEMI |
| 3+ | M | CHEMI |
| 3+ | S | GOLD |
| 3+ | S | GOLD |
| 3+ | VS | GOLD |
| 3+ | VS | GOLD |
| 3+ | VS | GOLD |
| 3+ | VS | GOLD |
| 2+ | S | CHEMI |
| 2+ | S | CHEMI |
| 2+ | S | CHEMI |
| 2+ | S | CHEMI |
| 2+ | S | GOLD |
| 2+ | S | GOLD |
| 2+ | S | GOLD |
| 2+ | S | GOLD |
| 2+ | S | GOLD |
| 1+ | S | GOLD |
| 1+ | VS | GOLD |
| 1+ | VW | GOLD |
| TRACE/1+ | M | CHEMI |
| TRACE | VS | CHEMI |
| TRACE | W | GOLD |
| TRACE | W | GOLD |
| TRACE | VS | GOLD |
| NEG | S | CHEMI |
| NEG | S | CHEMI |
| NEG | M | CHEMI |
| NEG | VW | GOLD |
| NEG | VW | GOLD |
| NEG | NEG | GOLD |
| NEG | NEG | GOLD |
| NEG | W | GOLD |
| NEG | W | GOLD |
| NEG | W | GOLD |

The hybridization results summarized in column 2 of Table 2 represent subjective interpretations of the intensity of hybridization signal obtained when labeled urine samples described in Table 1 were hybridized with genomic human DNA.

The "LEUKOSTIX"® assay is a colorimetric reagent strip assay. Color development on the reagent strip is compared to a chart provided with the assay reagent strips and ranges from negative (no color development) to 3+ (very strong color development).

Example 6: Lysis of Cells

A 1.0 mL aliquot of cell suspension was centrifuged and the cell pellet resuspended in 100 µL of unbuffered NaOH solution. The sample was then exposed to high temperature for a short time and then diluted to the original volume using 10 mM borate buffer. The pH of the solution was then adjusted to neutral with HCl.

Table 3 shows the efficiency of lysis of two different Gram positive cocci, Staphylococcus epidermidis and Streptococcus faecalis, at varying NaOH concentrations at either 68°C or 100°C. In this Example, the absorbance of the 10 mL aliquots at 600 nm was recorded before centrifugation. After centrifugation, the cell pellets were resuspended in varying concentrations of NaOH (100 µL) and duplicate samples of each exposed to 68°C for 10 minutes or 100°C for 5 minutes. Each sample was then diluted to 1.0 mL and the absorbance at 600 nm again recorded. Since the beginning and ending volumes are identical, the beginning and ending absorbance at 600 nm provides a direct measurement of lysis efficiency.

Whereas Gram negative organisms lysed efficiently in as low as 0.1 N NaOH, Table 3 shows clearly that efficient lysis is a function of both NaOH concentration and temperature, such that higher NaOH concentrations are required as the incubation temperature decreases. At 100°C (maximum temperature at 1 atmosphere) a concentration of at least 1.6 N NaOH was required for efficient lysis of S. epidermidis and S. faecalis. If lower temperatures are desirable or necessary, then higher concentrations of NaOH will be required to maintain lysis efficiency.

TABLE 3

EFFICIENCY OF LYSIS OF GRAM POSITIVE BACTERIA AT VARIOUS CONCENTRATIONS OF NaOH AT 68°C and 100°C

Streptococcus faecalis

| | 100°C/5 Minutes | | | 68°C/10 Minutes | | |
|---|---|---|---|---|---|---|
| [NaOH] | OD600 PRE | OD600 POST | %LYSIS | OD600 PRE | OD600 POST | %LYSIS |
| 0 N | 0.475 | 0.366 | 23 | 0.512 | 0.357 | 30 |
| 0.1 | .509 | .261 | 50 | .513 | .238 | 54 |
| 0.2 | .512 | .194 | 62 | .514 | .259 | 50 |
| 0.4 | .504 | .175 | 65 | .513 | .150 | 71 |
| 0.8 | .506 | .113 | 78 | .505 | .147 | 71 |
| 1.2 | .498 | .082 | 84 | .498 | .150 | 70 |
| 1.6 | .487 | .061 | 88 | .426 | .099 | 77 |

Staphylocuccus epidermidis

| | 100°C/5 Minutes | | | 68°C/10 Minutes | | |
|---|---|---|---|---|---|---|
| [NaOH] | OD600 PRE | OD600 POST | %LYSIS | OD600 PRE | OD600 POST | %LYSIS |
| 0 N | 0.667 | 0.558 | 16 | 0.690 | 0.560 | 19 |
| 0.1 | .681 | .396 | 42 | .701 | .441 | 37 |
| 0.2 | .674 | .296 | 60 | .699 | .414 | 41 |
| 0.4 | .699 | .183 | 74 | .730 | .309 | 58 |
| 0.8 | .705 | .091 | 87 | .715 | .187 | 74 |
| 1.2 | .680 | .070 | 90 | .719 | .090 | 88 |
| 1.6 | .693 | .035 | 95 | .660 | .040 | 94 |

Example 15: Direct Labeling of Nucleic Acids in an Infected Urine Sample

A set of clinical urine test samples was collected from a hospital. 1 ml of urine was deposited in a polypropylene tube (1.5 ml). To the urine 150µl of 8 N-sodium hydroxide solution in water was added. The mixture was maintained in a boiling water bath for five minutes. The alkaline suspension was then transferred to another tube containing 0.3 g of solid boric acid for neutralization. The mixture was shaken well by hand for mixing and solubilization. To this mixture, 10 microgram of the photolabeling reagent "bio-PEG-Ang", as used in Example 10 (i) was added (10 microliter of 1 mg/ml in water). The mixture was then irradiated for 60 minutes using a hand held UV lamp (UVL25) at a long wavelength setting. The tube was kept on ice during irradiation.

A nitrocellulose paper strip containing immobilized unlabeled genomic DNA probes (as used in Example 8), 700 microliter mixture containing 5% non-fat dry milk, 10% polyethylene glycol (MW-6000), 10mM sodium pyrophosphate, all in 3 x SSC and 300 µl labeled test samples were placed in a seal-a-meal bag. The bag was heat sealed. Hybridization was then conducted for two hours at 68°C by incubating the sealed bag in a water bath.

After hybridization, the nitrocellulose strip was washed at 68°C with 0.1 x SSC, 0.1% SDS for 30 minutes. The unsaturated sites were then blocked by immersing the paper in 3% BSA solution in 0.1 mM tris, 0.1 M sodium chloride, 2 mM magnesium chloride for 30 minutes. The hybrid was then detected by either immunogold, or by chemiluminescence or by the BRL method (See Examples 2, 3(a) or 3(b)). For every sample, the diagnosis was also done by bacteriological growth methods. The results were then compared for validity of the present method.

## Claims

**1.** A method for detecting one or more polynucleotide sequences from microorganisms or eucaryotic sources in a biological test sample, comprising the steps of:
(a) treating said biological test sample without prior centrifugation or filtration to lyse cells and to release nucleic acids therefrom,
(b) specifically labeling released sample nucleic acids directly in the lysate produced in step (a) without purification or isolation of specific nucleic acid sequences,
(c) contacting the resulting labeled nucleic acids, under hybridization conditions, with one or more immobilized oligonucleotide or nucleic acid probes hybridizable with the sequence or sequences to be detected, to thereby form hybridized labeled nucleic acids, and
(d) assaying for the hybridized nucleic acids by detecting the label, characterized in that the hybridization is accelerated by the addition of polyethylene glycol.

**2.** The method of claim 1 wherein the biological test sample is selected from urine, blood, cerebrospinal fluid, pus, amniotic fluid, tears, sputum, saliva, lung aspirate, vaginal discharge, stool, a solid tissue sample, a skin swab sample, a throat swab sample, and a genital swab sample.

**3.** The method of claim 1 or 2 wherein said labeling is performed by photochemically reacting a labeled form of a nucleic acid binding ligand with the sample nucleic acids.

**4.** The method of any one of claims 1 to 3 wherein the nucleic acids released from the sample are in double-stranded form and, prior to step (c), the labeled nucleic acids are denatured to provide labeled single-stranded nucleic acids.

**5.** The method of any one of claims 1 to 4 wherein said eucaryotic sources are selected from algae, protozoa, fungi, slime molds, and mammalian cells, or wherein said microorganisms are selected from Escherichia, Proteus, Klebsiella, Staphylococcus, Streptococcus, Pseudomonas and Lactobacillus.

**6.** The method of any one of claims 1 to 5 wherein cells in the sample are lysed by treatment with alkali.

**7.** The method of any one of claims 1 to 6 wherein the label is selected from protein binding ligands, haptens, antigens, fluorescent compounds, dyes, radioactive isotopes, and enzymes.

**8.** The method of any one of claims 1 to 7 wherein the probes are immobilized by covalent coupling or adsorption to a solid surface.

**9.** The method of any one of claims 1 to 8 wherein the probes are immobilized in the form of dots on a solid support strip.

## Patentansprüche

1. Verfahren zum Nachweis einer oder mehrerer Polynucleotid-Sequenzen aus Microorganismen oder eukaryontischen Quellen in einer biologischen Testprobe, worin Stufen enthalten sind, in denen man:
   (a) die genannte biologische Testprobe ohne vorherige Zentrifugation oder Filtration behandelt, um die Zellen zu lysieren und Nucleinsäuren daraus freizusetzen;
   (b) freigesetzte Proben-Nucleinsäuren direkt im Lysat spezifisch markiert, welches in Stufe (a) ohne Reinigung oder Isolierung spezifischer Nucleinsäure-Sequenzen erzeugt worden ist,
   (c) die sich ergebenden markierten Nucleinsäuren, unter Hybridisierungsbedingungen, mit einer oder mehreren immobilisierten Oligonucleotid- oder Nucleinsäure-Sonden in Kontakt bringt, die mit der oder den nachzuweisenden Sequenz(en) hybridisierbar sind, um dadurch hybridisierte markierte Nucleinsäuren zu bilden, und
   (d) die hybridisierten Nucleinsäuren mit einem Assayverfahren analysiert, indem man die Markierung nachweist,
   dadurch **gekennzeichnet**, daß
   die Hybridisierung durch Zugabe von Polyethylenglycol beschleunigt wird.

2. Verfahren gemäß Anspruch 1,
   worin die biologische Testprobe aus Urin, Blut, Cerebrospinal-Flüssigkeit, Eiter, amniotischer Flüssigkeit, Tränen, Auswurf, Speichel, Lungenaspirat, Vaginalausfluß, Stuhl, einer festen Gewebsprobe, einer Hautabstrichprobe, einer Rachenabstrichprobe und einer Genitalabstrichprobe ausgewählt ist.

3. Verfahren gemäß Anspruch 1 oder 2,
   worin die genannte Markierung durch photochemische Reaktion einer markierten Form eines Nucleinsäure-Bindungsligand mit den Proben-Nucleinsäuren durchgeführt wird.

4. Verfahren gemäß jedem der Ansprüche 1 bis 3,
   worin die aus der Probe freigesetzten Nucleinsäuren in doppelsträngiger Form vorliegen und, vor der Stufe (c), die markierten Nucleinsäuren denaturiert werden, um markierte einzelstrangige Nucleinsäuren zu ergeben.

5. Verfahren gemäß jedem der Ansprüche 1 bis 4,
   worin die genannten eukaryontischen Quellen aus Algen, Protozoen, Pilzen, Schleimmodern und Säugetierzellen oder die genannten Microorganismen aus Escherichia, Proteus, Klebsiella, Staphylococcus, Streptococcus, Pseudomonas und Lactobacillus ausgewählt sind.

6. Verfahren gemäß jedem der Ansprüche 1 bis 5,
   worin die Zellen in der Probe durch Behandlung mit Alkali lysiert werden.

7. Verfahren gemäß jedem der Ansprüche 1 bis 6,
   worin die Markierung aus Protein-Bindungsliganden, Haptenen, Antigenen, Fluoreszenzverbindungen, Farbstoffen, radioaktiven Isotopen und Enzymen ausgewählt ist.

8. Verfahren gemäß jedem der Ansprüche 1 bis 7,
   worin die Sonden durch kovalente Kupplung oder Adsorption an eine feste Oberfläche immobilisiert sind.

9. Verfahren gemäß jedem der Ansprüche 1 bis 8,
   worin die Sonden in der Form von Punkten auf einem festen Trägerunterlagenstreifen immobilisiert sind.

## Revendications

1. Méthode pour détecter une ou plusieurs séquences polynucléotidiques provenant de microorganismes ou de sources eucaryotiques dans un échantillon d'essai biologique, comprenant les étapes consistant :
   (a) à traiter ledit échantillon d'essai biologique, sans une centrifugation ou filtration préalable pour lyser les cellules et pour en libérer les acides nucléiques,

(b) à marquer spécifiquement les acides nucléiques libérés de l'échantillon directement dans le lysat produit dans l'étape (a) sans purification ou isolement des séquences d'acide nucléique spécifiques,
(c) à mettre en contact les acides nucléiques marqués résultants, dans des conditions d'hybridation, avec une ou plusieurs sondes d'oligonucléotides ou d'acides nucléiques immobilisées hybridables avec la ou les séquences à détecter, pour former ainsi des acides nucléiques marqués hybridés, et
(d) à analyser les acides nucléiques hybridés par détection du marqueur, caractérisée en ce que l'hybridation est accélérée par addition de polyéthylèneglycol.

**2.** Méthode suivant la revendication 1, dans laquelle l'échantillon d'essai biologique est choisi entre l'urine, le sang, le liquide céphalo-rachidien, le pus, le liquide amniotique, les larmes, des expectorations, la salive, un échantillon prélevé par aspiration pulmonaire, un produit d'écoulement vaginal, des selles, un échantillon de tissu solide, un prélèvement cutané par écouvillonage, un prélèvement de gorge par écouvillonage et un prélèvement génital par écouvillonage.

**3.** Méthode suivant la revendication 1 ou 2, dans laquelle le marquage est effectué en soumettant à une réaction photochimique une forme marquée d'un ligand de liaison aux acides nucléiques avec les acides nucléiques de l'échantillon.

**4.** Méthode suivant l'une quelconque des revendications 1 à 3, dans laquelle les acides nucléiques libérés de l'échantillon sont sous forme bicaténaire et, avant l'étape (c), les acides nucléiques marqués sont dénaturés pour fournir des acides nucléiques monocaténaires marqués.

**5.** Méthode suivant l'une quelconque des revendications 1 à 4, dans laquelle les sources eucaryotiques sont choisies entre des algues, des protozoaires, des champignons, des myxomycètes et des cellules de mammifères, ou dans laquelle les microorganismes sont choisis parmi les genres Escherichia, Proteus, Klebsiella, Stanhylococcus, Streptococcus, Pseudomonas et Lactobacillus.

**6.** Méthode suivant l'une quelconque des revendications 1 à 5, dans laquelle les cellules présentes dans l'échantillon sont lysées par traitement avec une substance alcaline.

**7.** Méthode suivant l'une quelconque des revendications 1 à 6, dans laquelle le marqueur est choisi entre des ligands de liaison aux protéines, des haptènes, des antigènes, des composés fluorescents, des colorants, des isotopes radioactifs et des enzymes.

**8.** Méthode suivant l'une quelconque des revendications 1 à 7, dans laquelle les sondes sont immobilisées par couplage covalent ou adsorption à une surface solide.

**9.** Méthode suivant l'une quelconque des revendications 1 à 8, dans laquelle les sondes sont immobilisées sous forme de points sur une bande solide de support.

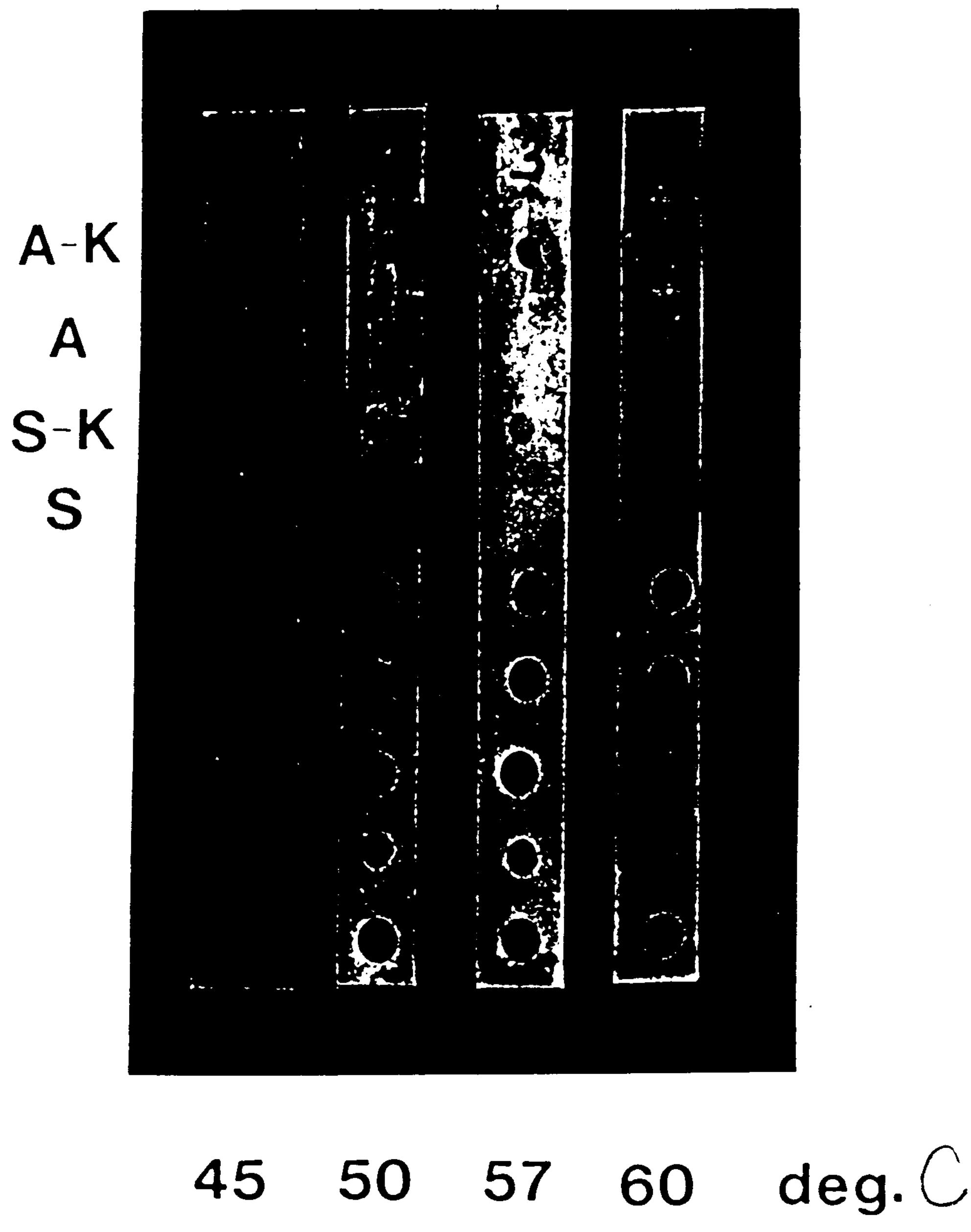


FIG. 1